# EUROPEAN PATENT SPECIFICATION

(11) **EP 2 854 747 B1**
(45) Date of publication and mention of the grant of the patent: **16.08.2017**
(21) Application number: 13723154.4
(22) Date of filing: 16.05.2013
(51) Int. Cl.: A61K 8/26, A61K 8/28, A61K 8/368, A61K 8/44, A61Q 15/00, A61K 8/06

(54) **COSMETIC COMPOSITION COMPRISING THE COMBINATION OF A LIPOPHILIC SALICYLIC ACID DERIVATIVE, AN ANTIPERSPIRANT ALUMINIUM SALT OR COMPLEX AND AN AMINO ACID-N,N-DIACETIC ACID SALT**
KOSMETISCHE ZUSAMMENSETZUNG ENTHALTEND EIN LIPOPHILES SALICYLSÄURE-DERIVAT, EIN ANTIPERSPIRANTES ALUMINIUMSALZ UND EIN N,N-DIESSIGSÄURE-AMINOSÄURE-SALZ
COMPOSITION COSMÉTIQUE COMPRENANT LA COMBINAISON D'UN DÉRIVÉ LIPOPHILE D'ACIDE SALICYLIQUE, D'UN SEL OU D'UN COMPLEXE D'ALUMINIUM ANTITRANSPIRANT ET D'UN SEL D'ACIDE (ACIDE AMINÉ)-N,N-DIACÉTIQUE

(30) Priority: 25.05.2012 FR 1254818; 23.07.2012 US 201261674608 P
(43) Date of publication of application: 08.04.2015
(73) Proprietor: L'Oréal, 75008 Paris (FR)
(72) Inventor: SEBILLOTTE-ARNAUD, Laurence, F-94240 L'hay les Roses (FR)
(74) Representative: Miszputen, Laurent
(86) International application number: PCT/EP2013/060199
(87) International publication number: WO 2013/174725

(56) References cited:
- EP-A1- 1 486 199
- FR-A1- 2 884 417
- US-A1- 2004 162 272
- Akzo Nobel: "Merit Award for GLDA chelates", , 8 July 2010 (2010-07-08), XP055059551, http://www.akzonobel.com/dissolvinegl/news /pressreleases/2010/20100708_merit_award_g lda_chelates.aspx Retrieved from the Internet: URL:http://www.akzonobel.com/dissolvinegl/ news/pressreleases/2010/20100708_merit_awa rd_glda_chelates.aspx [retrieved on 2013-04-15]

## Description

One subject of the invention is a cosmetic composition comprising, in a cosmetically acceptable medium, at least:
(a) a lipophilic salicylic acid derivative or a salt thereof of formula (I), the definition of which will be given below, and
(b) at least one antiperspirant aluminium salt or complex, and
(c) at least one sequestrant chosen from amino acid-N,N-diacetic acid salts.

The invention relates in particular to a composition in the form of an emulsion comprising said combination and at least one aqueous phase and at least one fatty phase, in particular an oil-in-water emulsion.

The invention also relates to a process for preparing a composition in the form of an emulsion, in particular an oil-in-water emulsion, as defined previously, characterized in that the amino acid-N,N-diacetic acid salt is introduced in the presence of the antiperspirant aluminium salt(s) or complex before mixing with the amino acid-N,N-diacetic acid salt and before emulsification of the aqueous phase and of the fatty phase.

The invention also relates to a process for the cosmetic treatment of human perspiration and optionally body odours, using this composition.

Eccrine or apocrine sweat has little odour when it is secreted. It is its degradation by bacteria via enzymatic reactions that produces malodorous compounds. Deodorant active agents have the function of reducing or preventing the formation of unpleasant odours. The various systems proposed hitherto may be grouped into major families:

### (i) Bactericidal substances or substances that limit bacterial growth:

There are bactericidal substances that destroy the resident bacterial flora. Among these substances, the one most widely used is Triclosan (2,4,4'-trichloro-2'-hydroxydiphenyl ether), which has the drawback of substantially modifying the ecology of the cutaneous flora. There are substances that reduce bacterial growth. Among these substances, mention may be made of transition metal chelating agents, for instance EDTA or DPTA. These materials deprive the medium of the metals required for the growth of the bacteria. These active agents are, unfortunately, potentially ecotoxic.

### (ii) Substances that block the enzymatic reactions responsible for the formation of odoriferous compounds:

Particular examples that may be mentioned include arylsulfatase inhibitors, 5-lipoxygenase inhibitors, aminoacylase inhibitors and β-glucuronidase inhibitors. Unfortunately, these inhibitors are often specific and thus are not very effective compared with antibacterial agents.

### (iii) Unpleasant-odour absorbers

These odour absorbers "capture" or reduce the volatility of odoriferous compounds. Odour absorbers that may be mentioned include zeolites and cyclodextrins. These compounds are difficult to formulate since the compounds of the formula may interact and reduce their efficacy. Eccrine sweat is secreted to enable thermolysis during body heat imbalances caused by effort or external heat. This sweat is responsible for the sensations of moisture and for the ring stains on clothing. Antiperspirant products were developed to prevent these unpleasant phenomena.

Several types of antiperspirant active agent exist:

### (a) Moisture absorbers:

The object of moisture absorbers is to capture sweat at the surface of the skin.
Perspiration exists, but the unpleasant phenomena associated therewith are avoided (disposable nappy principle). The moisture absorbers known in the prior art that may be mentioned include superabsorbent polymers of starch type grafted with homopolymers and copolymers of a sodium salt of poly(2-propenamide-co-propenoic acid) as described in patent application WO 03/030853.

### b) Film-forming agents:

The principle of film-forming agents is to form a film at the surface of the skin and thus prevent the sweat from being secreted.

### (c) Aluminium salts or complexes:

These active agents are the ones most commonly used as antiperspirant active agents. The principle of action of these active agents is considered to be the formation of a gel in the sweat duct. This gel creates a plug that partially blocks the sweat pores. The flow of sweat is thus reduced. These aluminium salts also have intrinsic efficacy since they are antibacterial agents. They thus also play a direct role on the deodorant efficacy by reducing the number of bacteria responsible for the degradation of sweat.

With the aim of obtaining long-term efficacy, there is a need to find better combinations of active agents.

The combination of a lipophilic salicylic acid derivative with an antiperspirant active agent is suggested in patent application WO 97/15278, which describes the cosmetic use of a substance P antagonist as an antiperspirant agent and the possibility of adding, as a lipophilic additive, a lipophilic salicylic acid derivative to these antiperspirant formulations.

The combination of a lipophilic salicylic acid derivative with an antiperspirant active agent has also been mentioned in patent application WO 04/073745. This document describes the efficacy-boosting effect of these lipophilic salicylic acid derivatives on 48 types of cosmetic active agent, among which are mentioned, without specifying their nature, antiperspirant active agents.

Among antiperspirant active agents, aluminium salts are cosmetic active agents that are generally used at contents of greater than 5% and preferably up to 20%. The Applicant has found that the addition of lipophilic salicylic acid derivatives as described in document WO 04/073745 to antiperspirant compositions based on aluminium salts could disrupt the stability of these formulations and in particular strongly colour the products obtained, making them unsuitable for use.

In patent EP 1 877 033B1, a deodorant product with long-term efficacy, which is stable on storage and in which the colouring phenomenon is reduced was proposed by combining (a) at least one particular lipophilic salicylic acid derivative of formula (I) as defined later with (b) at least one antiperspirant aluminium salt in an (a)/(b) weight ratio of less than 1/20, preferably less than 1/25 and more preferably still less than 1/50. Specifically, the combination of active agents used in a ratio of greater than 1/25 could destabilize the formulation containing them and/or produce a pink colour that is undesirable for the user. However, this type of composition limits the possibilities of using this combination at particular ratios and may also result in a pink colouration during storage.

There remains a need to find a deodorant cosmetic composition comprising:
(a) at least one lipophilic salicylic acid derivative or a salt thereof and (b) at least one antiperspirant aluminium salt or complex without being limited to salicylic derivative/antiperspirant salt weight ratios of less than 1/20, in which the phenomenon of colouring of the composition during storage is not observed or is only very slightly detected.

The Applicant has surprisingly discovered that by adding a sequestrant chosen from amino acid-N,N-diacetic acid salts of formula (I), the definition of which will be given below, to the lipophilic salicylic acid derivative/aluminium salt or complex combination, the stability of the composition on storage was substantially improved, namely that the phenomenon of colouring of the composition during storage was substantially eliminated or reduced.

One subject of the invention is a cosmetic composition comprising, in a cosmetically acceptable medium, at least:
(a) a lipophilic salicylic acid derivative or a salt thereof, and
(b) at least one antiperspirant aluminium salt or complex, and
(c) at least one sequestrant chosen from amino acid-N,N-diacetic acid salts of formula (I), the definition of which will be given below.

The invention relates in particular to a composition in the form of an emulsion comprising said combination and at least one aqueous phase and at least one fatty phase, in particular an oil-in-water emulsion.

The invention also relates to a process for preparing a composition in the form of an emulsion, in particular an oil-in-water emulsion, as defined previously, characterized in that the amino acid-N,N-diacetic acid salt is introduced in the presence of the antiperspirant aluminium salt(s) or complex(es) before mixing with the amino acid-N,N-diacetic acid salt and before emulsification of the aqueous phase and of the fatty phase.

The invention also relates to a process for the cosmetic treatment of human perspiration and optionally body odours, using this composition.

Within the meaning of the present invention, the expression "cosmetically acceptable medium" is understood to mean a medium which is suitable for the topical administration of a composition. A physiologically acceptable medium is preferably a cosmetically or dermatologically acceptable medium, that is to say a medium which is devoid of unpleasant odour or appearance and which is entirely compatible with the topical administration route. In the present case, where the composition is intended for topical administration, that is to say for administration by application at the surface of the keratinous substance under consideration, such a medium is considered in particular to be physiologically acceptable when it does not cause stinging, tightness or redness unacceptable to the user.

The expression "antiperspirant aluminium salt or complex" is understood to mean any salt or any complex of aluminium which, by itself alone, has the effect of reducing the flow of sweat, of reducing the sensation on the skin of moisture associated with human sweat and of masking human sweat.

The expression "lipophilic" salicylic acid derivative is understood to mean any salicylic acid derivative that is not soluble at 1% by weight in water and at 25°C.

The term "sequestrant" is understood to mean any substance capable of forming a complex with at least one metal (alkali metal, alkaline-earth metal or transition metal) ion.

### LIPOPHILIC SALICYLIC ACID DERIVATIVES

The lipophilic salicylic acid derivatives in accordance with the invention correspond to the formula (I): 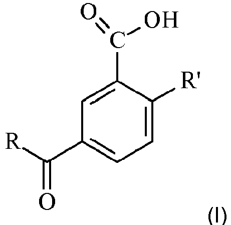 in which:
- the R radical denotes a linear, branched or cyclic, saturated aliphatic chain containing from 2 to 22 carbon atoms; an unsaturated chain containing from 2 to 22 carbon atoms and comprising one or more double bonds that may be conjugated; an aromatic ring bonded to the carbonyl radical directly or via saturated or unsaturated aliphatic chains containing from 2 to 7 carbon atoms; it being possible for said groups to be substituted with one or more substituents, which may be identical or different, chosen from (a) halogen atoms, (b) a trifluoromethyl group, (c) hydroxyl groups in free form or esterified with an acid containing from 1 to 6 carbon atoms, or (d) a carboxyl function in free form or esterified with a lower alcohol containing from 1 to 6 carbon atoms;
- R' is a hydroxyl group or an ester group of formula: 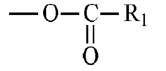 in which R₁ denotes a linear or branched, saturated or unsaturated aliphatic chain containing from 1 to 18 carbon atoms;
- and also salts thereof derived from a mineral or organic base.

The lipophilic salicylic acid derivatives of formula (I) that may be used according to the invention are described in patents US 6,159,479 and US 5,558,871, FR 2,581,542, US 4,767,750, EP 378 936, US 5,267,407, US 5,667,789, US 5,580,549 and EP-A-570,230.

Preferentially, the R radical denotes a linear, branched or cyclic, saturated aliphatic chain containing from 3 to 11 carbon atoms; an unsaturated chain containing from 3 to 17 carbon atoms and comprising one or more conjugated or unconjugated double bonds; it being possible for said hydrocarbon-based chains to be substituted with one or more substituents, which may be identical or different, chosen from (a) halogen atoms, (b) a trifluoromethyl group, (c) hydroxyl groups in free form or esterified with an acid containing from 1 to 6 carbon atoms, or (d) a carboxyl function in free form or esterified with a lower alcohol containing from 1 to 6 carbon atoms;
- R' is a hydroxyl group or an ester group of formula: 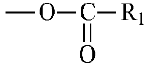 in which R₁ denotes a radical -O-(C=O)-(CH₂)ₙ-CH₃ where n is a number ranging from 0 to 14;
- and also salts thereof obtained by salification with a mineral or organic base.

The compounds that are more particularly preferred are those in which the R radical is a C₃-C₁₁ alkyl group and R' denotes hydroxyl.

Other particularly advantageous compounds are those in which R represents a chain derived from linoleic, linolenic or oleic acid.

Another group of particularly preferred compounds is constituted of compounds in which the R radical denotes a C₃-C₁₁ alkyl group bearing a carboxyl function in free form or esterified with a lower alcohol containing from 1 to 6 carbon atoms and R' denotes hydroxyl.

The salts of the lipophilic salicylic acid derivatives of formula (I) may be obtained by salification with a mineral or organic base. Examples of mineral bases that may be mentioned include alkali metal hydroxides, for instance sodium hydroxide (caustic soda) or potassium hydroxide (caustic potash), alkaline-earth metal hydroxides or aqueous ammonia.

Among the organic bases that may be mentioned are amines and alkanolamines. Quaternary salts, for instance those described in patent FR 2 607 498, are particularly advantageous.

The lipophilic salicylic acid derivatives of formula (I) that may be used according to the invention are described in patents US 6,159,479 and US 5,558,871, FR 2,581,542, FR 2 607 498, US 4,767,750, EP 378,936, US 5,267,407, US 5,667,789, US 5,580,549 and EP-A-570,230.

Among the compounds of formula (I) that are particularly preferred, mention may be made of:
5-n-octanoylsalicylic acid (or capryloyl salicylic acid); 5-n-decanoylsalicylic acid; 5-n-dodecanoyl-salicylic acid; 5-n-heptyloxysalicylic acid and the corresponding salts thereof.

Use will more particularly be made of 5-n-octanoylsalicylic acid (or capryloyl salicylic acid) manufactured under the trade name Mexoryl SAB by the company CHIMEX (see page 139 of the document International Cosmetic Ingredient Dictionary, 6th Edition, Volume 1, published by the review Cosmetic, Toiletry, and Fragrance Association, 1995).

In the compositions of the invention, the concentration of salicylic compound of formula (I) preferably ranges from 0.001% to 20% by weight, more preferentially from 0.01% to 15% by weight and more preferably still from 0.05% to 5% by weight relative to the total weight of the composition.

### ANTIPERSPIRANT ALUMINIUM SALTS OR COMPLEXES

The antiperspirant active agents in accordance with the invention are preferably chosen from aluminium salts; complexes of zirconium hydroxychloride and of aluminium hydroxychloride with an amino acid, such as those described in patent US-3 792 068, commonly known as "ZAG" complexes. Such complexes are generally known under the name ZAG (when the amino acid is glycine). The ZAG complexes ordinarily exhibit an Al/Zr quotient ranging from approximately 1.67 to 12.5 and a metal/Cl quotient ranging from approximately 0.73 to 1.93. Mention may be made, among these products, of aluminium zirconium octachlorohydrex GLY, aluminium zirconium pentachlorohydrex GLY, aluminium zirconium tetrachlorohydrate GLY and aluminium zirconium trichlorohydrate GLY.

Mention may be made, among the aluminium salts, of aluminium chlorohydrate, aluminium chlorohydrex, aluminium chlorohydrex PEG, aluminium chlorohydrex PG, aluminium dichlorohydrate, aluminium dichlorohydrex PEG, aluminium dichlorohydrex PG, aluminium sesquichlorohydrate, aluminium sesquichlorohydrex PEG, aluminium sesquichlorohydrex PG, alum salts, aluminium sulfate, aluminium zirconium octachlorohydrate, aluminium zirconium pentachlorohydrate, aluminium zirconium tetrachlorohydrate, aluminium zirconium trichlorohydrate and more particularly the aluminium hydroxychloride sold by the company Reheis under the name Reach 301 or by the company Guilini Chemie under the name Aloxicoll PF 40. Aluminium zirconium salts are, for example, the salt sold by the company Reheis under the name Reach AZP-908-SUF.

Use will more particularly be made of aluminium chlorohydrate in activated or non-activated form.

The antiperspirant active agents in accordance with the invention may be present in the composition according to the invention in a proportion of from 0.001% to 30% by weight and preferably in a proportion of from 0.5% to 25% by weight relative to the total weight of the composition.

### AMINO ACID-N,N-DIACETIC ACID SALTS

In the chemical structure of the sequestrants of amino acid-N,N-diacetic acid salt type in accordance with the invention, the amino acid-N,N-diacetic acid generally corresponds to the formula (II) below:

A-N-(CH₂COOH)₂ (II)

in which:
ANH₂ denotes an amino acid and -N- represents an amino group in the amino acid.

Among the amino acid-N,N-diacetic acids present in the chemical structure of the sequestrants in accordance with the invention, mention may be made of:
- glutamic acid-N,N-diacetic acid of the following structure: 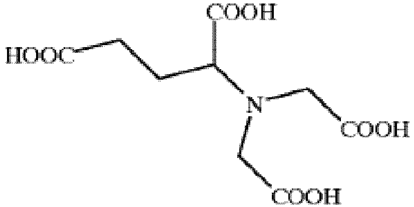
- α-alanine-N,N-diacetic acid of the following structure: 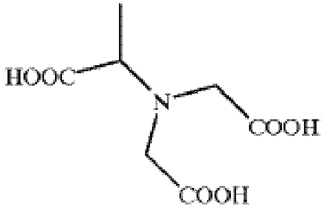
- β-alanine-N,N-diacetic acid of the following structure: 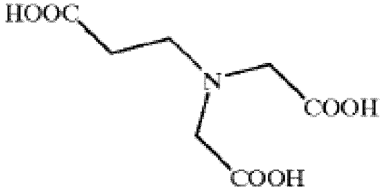
- aspartic acid-N,N-diacetic acid of the following structure: 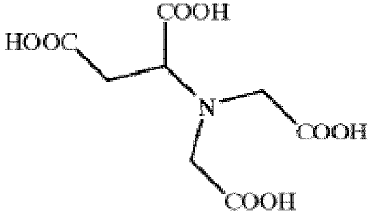
- glycine-N,N-diacetic acid of the following structure: 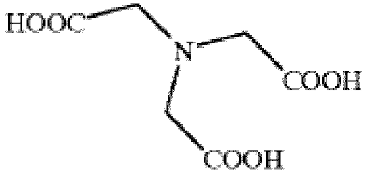
- serine-N,N-diacetic acid of the following structure:

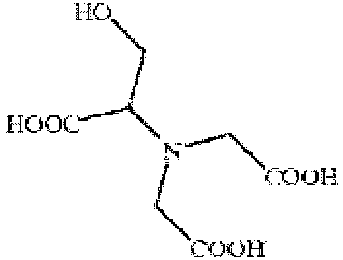

Use will more particularly be made of glutamic acid-N,N-diacetic acid.

These acids may be synthesized by the known Strecker reaction using an amino acid as starting material, or may be prepared from a monochloroacetic acid.

In the chemical structure of the amino acid-N,N-diacetic acid salts in accordance with the invention, all the carboxylic functions borne by the amino acid and the diacetic acid are salified with a mineral base and/or an organic base.

Examples of mineral bases that may be mentioned include alkali metal hydroxides, for instance sodium hydroxide (caustic soda) or potassium hydroxide (caustic potash), alkaline-earth metal hydroxides or aqueous ammonia.

The organic base may be chosen, in particular, from primary, secondary or tertiary amines, in particular alkanolamines such as monoethanolamine, triethanolamine or diethanolamine.

Use will more particularly be made of amino acid-N,N-diacetic acid sodium salts in which all the carboxylic functions are in sodium salt form.

Use will more particularly be made of glutamic acid-N,N-diacetic acid tetrasodium salt, having the INCl name: TETRASODIUM GLUTAMATE DIACETATE, such as the commercial product sold under the name Dissolvine GL-47-S ® by the company Akzo Nobel.

The amino acid-N,N-diacetic acid salts in accordance with the invention may be present in the composition according to the invention in a proportion of from 0.05% to 1% by weight of active material and preferably in a proportion of from 0.3% to 0.5% by weight of active material relative to the total weight of the composition.

### FORMULATION FORMS

The composition according to the invention can be provided in any formulation form conventionally used for a topical application and in particular in the form of aqueous gels or of aqueous or aqueous/alcoholic solutions. They can also, by addition of a fatty or oily phase, be provided in the form of dispersions of the lotion type, of emulsions with a liquid or semi-liquid consistency of the milk type, obtained by dispersion of a fatty phase in an aqueous phase (O/W) or vice versa (W/O), or of suspensions or emulsions with a soft, semi-solid or solid consistency of the cream or gel type, or alternatively of multiple emulsions (W/O/W or O/W/O), of microemulsions, of vesicular dispersions of ionic and/or nonionic type, or of wax/aqueous phase dispersions. These compositions are prepared according to the usual methods.

The compositions may especially be packaged in pressurized form in an aerosol device or in a pump dispenser; packaged in a device equipped with an openwork wall, especially a grille; packaged in a device equipped with a ball applicator ("roll-on"); packaged in the form of sticks or in the form of loose or compacted powder. In this regard, they contain the ingredients generally used in products of this type, which are well known to those skilled in the art.

According to another particular form of the invention, the compositions according to the invention may be anhydrous.

The term "anhydrous composition" means a composition containing less than 2% by weight of water, indeed even less than 0.5% of water, and especially devoid of water, the water not being added during the preparation of the composition but corresponding to the residual water contributed by the mixed ingredients.

According to another particular form of the invention, the compositions according to the invention may be solid, in particular in wand or stick form.

The term "solid composition" means that the measurement of the maximum force measured by texturometry during the penetration of a probe into the sample of formula must be at least equal to 0.25 newtons, in particular at least equal to 0.30 newtons and especially at least equal to 0.35 newtons, assessed under precise measurement conditions as follows.

The formulae are poured hot into jars 4 cm in diameter and 3 cm deep. Cooling is performed at ambient temperature. The hardness of the formulae produced is measured after an interval of 24 hours. The jars containing the samples are characterized in texturometry using a texture analyser such as the machine sold by the company Rheo TA-XT2, according to the following protocol: a stainless-steel ball probe 5 mm in diameter is brought into contact with the sample at a speed of 1 mm/s. The measurement system detects the interface with the sample, with a detection threshold equal to 0.005 newtons. The probe penetrates 0.3 mm into the sample, at a speed of 0.1 mm/s. The measuring machine records the change in force measured in compression over time, during the penetration phase. The hardness of the sample corresponds to the average of the maximum force values detected during penetration, over at least three measurements.

According to one particularly preferred form of the invention, the composition of the invention is provided in the form of an emulsion comprising at least one aqueous phase and at least one fatty phase and more particularly in the form of an oil-in-water emulsion.

Preferably, the composition in the form of an emotion will be prepared according to a process in which the amino acid-N,N-diacetic acid salt is introduced in the presence of the antiperspirant aluminium salt(s) or complex(es) before mixing with the amino acid-N,N-diacetic acid salt and before emulsification of the aqueous phase and of the fatty phase. By this process, a better stability on storage of the composition is observed, which is expressed in particular by the absence of colouration or only very slight colouration.

### AQUEOUS PHASE

The compositions according to the invention intended for cosmetic use may comprise at least one aqueous phase. They are especially formulated as aqueous lotions or as water-in-oil or oil-in-water emulsions or as multiple emulsions (oil-in-water-in-oil or water-in-oil-in-water triple emulsions (such emulsions are known and described, for example, by C. Fox in "Cosmetics and Toiletries" - November 1986 - Vol. 101 - pages 101-112)).

The aqueous phase of said compositions contains water and generally other water-soluble or water-miscible solvents. The water-soluble or water-miscible solvents comprise short chain, for example C₁-C₄, monoalcohols, such as ethanol or isopropanol; diols or polyols, for instance ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether and sorbitol. Propylene glycol and glycerol and propane-1,3-diol, will be used more particularly.

The composition according to the invention preferably has a pH ranging from 3 to 9 depending on the chosen support.

### EMULSIFIERS

### Oil-in-water emulsifiers

As emulsifiers that may be used in the oil-in-water emulsions or oil-in-water-in-oil triple emulsions, examples that may be mentioned include nonionic emulsifiers such as oxyalkylenated (more particularly polyoxyethylenated) fatty acid esters of glycerol; oxyalkylenated fatty acid esters of sorbitan; oxyalkylenated (oxyethylenated and/or oxypropylenated) fatty acid esters; oxyalkylenated (oxyethylenated and/or oxypropylenated) fatty alcohol ethers; sugar esters such as sucrose stearate; and mixtures thereof, such as the mixture of glyceryl stearate and PEG-40 stearate.

Mention may also be made of fatty alcohol/alkyl polyglycoside emulsifying mixtures as described in patent applications WO 92/06778, WO 95/13863 and WO 98/47610, for instance the commercial products sold by the company SEPPIC under the name Montanov ®.

### Water-in-oil emulsifiers

Among the emulsifiers that may be used in the water-in-oil emulsions or water-in-oil-in-water triple emulsions or triple emulsions, examples that may be mentioned include alkyl dimethicone copolyols corresponding to formula (III) below: 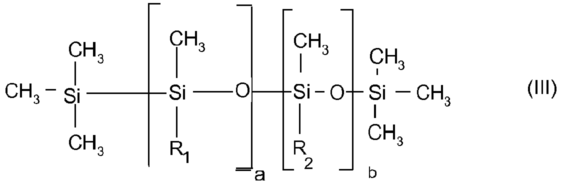 in which:
R₁ denotes a linear or branched C₁₂-C₂₀ and preferably C₁₂-C₁₈ alkyl group;
R₂ denotes the group: --CₙH₂ₙ--(-OC₂H₄-)ₓ--(-OC₃H₆-)_{y}--O-R₃;
R₃ denotes a hydrogen atom or a linear or branched alkyl radical comprising from 1 to 12 carbon atoms;
a is an integer ranging from 1 to about 500;
b denotes an integer ranging from 1 to about 500;
n is an integer ranging from 2 to 12 and preferably from 2 to 5;
x denotes an integer ranging from 1 to about 50 and preferably from 1 to 30;
y denotes an integer ranging from 0 to about 49 and preferably from 0 to 29, with the proviso that, when y is other than zero, the ratio x/y is greater than 1 and preferably varies from 2 to 11.

Among the alkyl dimethicone copolyol emulsifiers of formula (III) that are preferred, mention will be made more particularly of Cetyl PEG/PPG-10/1 Dimethicone and more particularly the mixture Cetyl PEG/PPG-10/1 Dimethicone and Dimethicone (INCl name), for instance the product sold under the trade name Abil EM90 by the company Goldschmidt, or alternatively the mixture (Polyglyceryl-4 Stearate and Cetyl PEG/PPG-10 (and) Dimethicone (and) Hexyl Laurate), for instance the product sold under the trade name Abil WE09 by the same company.

Among the water-in-oil emulsifiers, mention may also be made of the dimethicone copolyols corresponding to formula (IV) below: 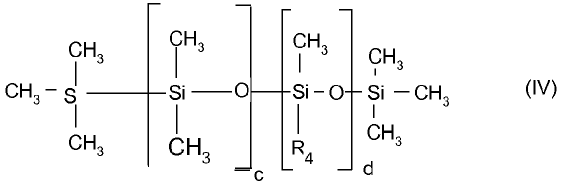 in which:
R₄ denotes the group: --CₘH₂ₘ--(-OC₂H₄-)ₛ-(-OC₃H₆-)t--O-R₅;
R₅ denotes a hydrogen atom or a linear or branched alkyl radical comprising from 1 to 12 carbon atoms;
c is an integer ranging from 1 to about 500;
d denotes an integer ranging from 1 to about 500;
m is an integer ranging from 2 to 12 and preferably from 2 to 5;
s denotes an integer ranging from 1 to about 50 and preferably from 1 to 30;
t denotes an integer ranging from 0 to about 50 and preferably from 0 to 30; with the proviso that the sum s+t is greater than or equal to 1.

Among these preferential dimethicone copolyol emulsifiers of formula (IV), use will particularly be made of PEG-18/PPG-18 Dimethicone and more particularly the mixture Cyclopentasiloxane (and) PEG-18/PPG-18 Dimethicone (INCl name), such as the product sold by the company Dow Corning under the trade name Silicone DC 5225 C or KF-6040 from the company Shin Etsu.

According to a particularly preferred form, use will be made of a mixture of at least one emulsifier of formula (I) and of at least one emulsifier of formula (IV).

Use will be made more particularly of a mixture of PEG-18/PPG-18 Dimethicone and Cetyl PEG/PPG-10/1 Dimethicone and even more particularly of a mixture of (Cyclopentasiloxane (and) PEG-18/PPG-18 Dimethicone) and of Cetyl PEG/PPG-10/1 Dimethicone and Dimethicone or of (Polyglyceryl-4 Stearate and Cetyl PEG/PPG-10 (and) Dimethicone (and) Hexyl Laurate).

Among the water-in-oil emulsifiers, mention may also be made of nonionic emulsifiers derived from fatty acids and polyols, alkyl polyglycosides (APGs), sugar esters and mixtures thereof.

As nonionic emulsifiers derived from fatty acids and polyols, use may be made especially of fatty acid esters of polyols, the fatty acid especially containing a C₈-C₂₄ alkyl chain, and the polyols being, for example, glycerol and sorbitan.

Fatty acid esters of polyols that may especially be mentioned include isostearic acid esters of polyols, stearic acid esters of polyols, and mixtures thereof, in particular isostearic acid esters of glycerol and/or sorbitan.

Stearic acid esters of polyols that may especially be mentioned include the polyethylene glycol esters, for instance PEG-30 Dipolyhydroxystearate, such as the product sold under the name Arlacel P135 by the company ICl.

Glycerol and/or sorbitan esters that may be mentioned, for example, include polyglyceryl isostearate, such as the product sold under the name Isolan GI 34 by the company Goldschmidt; sorbitan isostearate, such as the product sold under the name Arlacel 987 by the company ICl; sorbitan glyceryl isostearate, such as the product sold under the name Arlacel 986 by the company ICl, the mixture of sorbitan isostearate and polyglyceryl isostearate (3 mol) sold under the name Arlacel 1690 by the company Uniqema, and mixtures thereof.

The emulsifier may also be chosen from alkyl polyglycosides with an HLB of less than 7, for example those represented by the general formula (V) below:

R-O-(G)ₓ (V)

in which R represents a branched and/or unsaturated alkyl radical comprising from 14 to 24 carbon atoms, G represents a reduced sugar comprising 5 or 6 carbon atoms, and x is a value ranging from 1 to 10 and preferably from 1 to 4, and G especially denotes glucose, fructose or galactose.

The unsaturated alkyl radical may comprise one or more ethylenically unsaturated groups, and in particular one or two ethylenically unsaturated groups.

As alkyl polyglycosides of this type, mention may be made of alkyl polyglucosides (G = glucose in formula (V)), and especially the compounds of formula (V) in which R more particularly represents an oleyl radical (unsaturated C₁₈ radical) or isostearyl radical (saturated C₁₈ radical), G denotes glucose, x is a value ranging from 1 to 2, especially isostearyl glucoside or oleyl glucoside, and mixtures thereof. This alkyl polyglucoside may be used as a mixture with a coemulsifier, more especially with a fatty alcohol and especially a fatty alcohol containing the same fatty chain as that of the alkyl polyglucoside, i.e. comprising from 14 to 24 carbon atoms and containing a branched and/or unsaturated chain, and for example isostearyl alcohol when the alkyl polyglucoside is isostearyl glucoside, and oleyl alcohol when the alkyl polyglucoside is oleyl glucoside, optionally in the form of a self-emulsifying composition, as described, for example, in the document WO-A-92/06778. Use may be made, for example, of the mixture of isostearyl glucoside and isostearyl alcohol, sold under the name Montanov WO 18 by the company SEPPIC, and also the mixture of octyldodecanol and octyldodecyl xyloside sold under the name Fludanov 20X by the company SEPPIC.

Mention may also be made of succinic-terminated polyolefins, for instance esterified succinic-terminated polyisobutylenes and salts thereof, especially the diethanolamine salts, such as the products sold under the names Lubrizol 2724, Lubrizol 2722 and Lubrizol 5603 by the company Lubrizol or the commercial product Chemcinnate 2000.

The total amount of emulsifiers in the composition will preferably be, in the composition according to the invention, at active material contents ranging from 1% to 8% by weight and more particularly from 2% to 6% by weight, relative to the total weight of the composition.

### FATTY PHASE

The compositions according to the invention may contain at least one water-immiscible organic liquid phase, known as a fatty phase. This phase generally comprises one or more hydrophobic compounds that render said phase water-immiscible. Said phase is liquid (in the absence of structuring agent) at ambient temperature (20-25°C). Preferentially, the water-immiscible organic liquid phase in accordance with the invention is generally constituted of at least one volatile oil and/or one non-volatile oil and optionally at least one structuring agent.

The term "oil" means a fatty substance that is liquid at ambient temperature (25°C) and atmospheric pressure (760 mmHg, i.e. 105 Pa). The oil may be volatile or non-volatile.

For the purposes of the invention, the term "volatile oil" means an oil that is capable of evaporating on contact with the skin or the keratin fibre in less than one hour, at ambient temperature and atmospheric pressure. The volatile oils of the invention are volatile cosmetic oils, which are liquid at ambient temperature, having a non-zero vapour pressure, at ambient temperature and atmospheric pressure, ranging in particular from 0.13 Pa to 40 000 Pa (10⁻³ to 300 mmHg), in particular ranging from 1.3 Pa to 13 000 Pa (0.01 to 100 mmHg), and more particularly ranging from 1.3 Pa to 1300 Pa (0.01 to 10 mmHg).

The term "non-volatile oil" means an oil that remains on the skin or the keratin fibre at ambient temperature and atmospheric pressure for at least several hours, and that especially has a vapour pressure of less than 10⁻³ mmHg (0.13 Pa).

The oil may be chosen from any physiologically acceptable oil and in particular cosmetically acceptable oils, especially mineral, animal, vegetable or synthetic oils; in particular volatile or non-volatile hydrocarbon-based oils and/or silicone oils and/or fluoro oils, and mixtures thereof.

More precisely, the term "hydrocarbon-based oil" means an oil mainly comprising carbon and hydrogen atoms and optionally one or more functional groups chosen from hydroxyl, ester, ether and carboxylic functional groups. Generally, the oil has a viscosity of from 0.5 to 100 000 mPa.s, preferably from 50 to 50 000 mPa.s and more preferably from 100 to 300 000 mPa.s.

As examples of volatile oils that may be used in the invention, mention may be made of:
- volatile hydrocarbon-based oils chosen from hydrocarbon-based oils containing from 8 to 16 carbon atoms, and especially C₈-C₁₆ isoalkanes of petroleum origin (also known as isoparaffins), for instance isododecane (also known as 2,2,4,4,6-pentamethylheptane), isodecane and isohexadecane, for example the oils sold under the trade names Isopar or Permetyl, branched C₈-C₁₆ esters and isohexyl neopentanoate, and mixtures thereof. Use may also be made of other volatile hydrocarbon-based oils, for instance petroleum distillates, especially those sold under the name Shell Solt by the company Shell; and volatile linear alkanes, such as those described in patent application DE10 2008 012 457 from the company Cognis;
- volatile silicones, for instance volatile linear or cyclic silicone oils, especially those with a viscosity ≤ 8 centistokes (8×10⁻6 m²/s) and especially containing from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane or dodecamethylpentasiloxane;

- and mixtures thereof.

Mention may also be made of the volatile linear alkyltrisiloxane oils of general formula (VI): 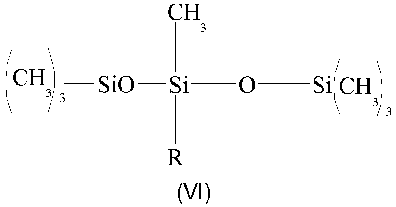 where R represents an alkyl group comprising from 2 to 4 carbon atoms, one or more hydrogen atoms of which can be substituted by a fluorine or chlorine atom.

Mention may be made, among the oils of general formula (I), of:
3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (I) for which R is, respectively, a butyl group, a propyl group or an ethyl group.

As examples of non-volatile oils that may be used in the invention, mention may be made of:
- hydrocarbon-based oils of animal origin, such as perhydrosqualene;
- hydrocarbon-based plant oils such as liquid triglycerides of fatty acids having 4 to 24 carbon atoms, for instance heptanoic or octanoic acid triglycerides, or else wheatgerm oil, olive oil, sweet almond oil, palm oil, rapeseed oil, cottonseed oil, alfalfa oil, poppyseed oil, pumpkin oil, marrow oil, blackcurrant oil, evening primrose oil, millet oil, barley oil, quinoa oil, rye oil, safflower oil, candlenut oil, passion flower oil, musk rose oil, sunflower oil, corn oil, soybean oil, marrow oil, grapeseed oil, sesame seed oil, hazelnut oil, apricot oil, macadamia oil, castor oil, avocado oil, caprylic/capric acid triglycerides, for instance those sold by the company Stéarineries Dubois or those sold under the names Miglyol 810, 812 and 818 by the company Dynamit Nobel, jojoba oil and shea butter oil;
- linear or branched hydrocarbons, of mineral or synthetic origin, such as liquid paraffins and derivatives thereof, petroleum jelly, polydecenes, polybutenes, hydrogenated polyisobutene such as Parleam, or squalane;
- synthetic ethers containing from 10 to 40 carbon atoms;
- synthetic esters, especially of fatty acids, for instance the oils of formula R₁COOR₂ in which R₁ represents a linear or branched higher fatty acid residue containing from 1 to 40 carbon atoms and R₂ represents a hydrocarbon-based chain, which is especially branched, containing from 1 to 40 carbon atoms, with R₁ + R₂ ≥ 10, for instance Purcellin oil (cetostearyl octanoate), isononyl isononanoate, isopropyl myristate, isopropyl palmitate, C₁₂-C₁₅ alcohol benzoate, hexyl laurate, diisopropyl adipate, isononyl isononanoate, 2-ethylhexyl palmitate, 2-octyldodecyl stearate, 2-octyldodecyl erucate, isostearyl isostearate or tridecyl trimellitate; alcohol or polyalcohol octanoates, decanoates or ricinoleates, for instance propylene glycol dioctanoate; hydroxylated esters, for instance isostearyl lactate, octyl hydroxystearate, octyldodecyl hydroxystearate, diisostearyl malate, triisocetyl citrate, and fatty alcohol heptanoates, octanoates or decanoates; polyol esters, for instance propylene glycol dioctanoate, neopentyl glycol diheptanoate or diethylene glycol diisononanoate; and pentaerythritol esters, for instance pentaerythrityl tetraisostearate;
- fatty alcohols that are liquid at ambient temperature, containing a branched and/or unsaturated carbon-based chain containing from 12 to 26 carbon atoms, for instance octyldodecanol, isostearyl alcohol, 2-butyloctanol, 2-hexyldecanol, 2-undecylpentadecanol or oleyl alcohol;
- higher fatty acids such as oleic acid, linoleic acid or linolenic acid;
- carbonates;
- acetates;
- citrates;
- fluoro oils that are optionally partially hydrocarbon-based and/or silicone-based, for instance fluorosilicone oils, fluoro polyethers and fluorosilicones as described in the document EP-A-847 752;
- silicone oils, for instance non-volatile linear or cyclic polydimethylsiloxanes (PDMSs); polydimethylsiloxanes comprising alkyl, alkoxy or phenyl groups, which are pendent or at the end of a silicone chain, these groups containing from 2 to 24 carbon atoms; phenyl silicones, for instance phenyl trimethicones, phenyl dimethicones, phenyl trimethylsiloxy diphenyl siloxanes, diphenyl dimethicones, diphenyl methyldiphenyl trisiloxanes and 2-phenylethyl trimethylsiloxy silicates, and
- mixtures thereof.

### STRUCTURING AGENT

The compositions according to the invention comprising a fatty phase may also contain at least one agent for structuring said fatty phase, which may preferably be chosen from waxes, pasty compounds, and mineral or organic lipophilic gelling agents, and mixtures thereof.

It is understood that the amount of these compounds may be adjusted by a person skilled in the art so as not to harm the effect desired in the context of the present invention.

### Wax(es)

The wax is in general a lipophilic compound that is solid at ambient temperature (25°C), with a reversible solid/liquid change in state, having a melting point of greater than or equal to 30°C, which may be up to 200°C and in particular up to 120°C.

In particular, the waxes suitable for the invention can exhibit a melting point of greater than or equal to 45°C and in particular of greater than or equal to 55°C.

For the purposes of the invention, the melting point corresponds to the temperature of the most endothermic peak observed in thermal analysis (DSC) as described in the standard ISO 11357-3; 1999. The melting point of the wax may be measured using a differential scanning calorimeter (DSC), for example the calorimeter sold under the name MDSC 2920 by the company TA Instruments.

The measuring protocol is as follows:
A sample of 5 mg of wax placed in a crucible is subjected to a first temperature rise ranging from -20°C to 100°C, at a heating rate of 10°C/minute, it is then cooled from 100°C to -20°C at a cooling rate of 10°C/minute and it is finally subjected to a second temperature rise ranging from -20°C to 100°C at a heating rate of 5°C/minute. During the second temperature rise, the variation in the difference in power absorbed by the empty crucible and by the crucible containing the sample of wax is measured as a function of the temperature. The melting point of the compound is the value of the temperature corresponding to the tip of the peak of the curve representing the variation in the difference in power absorbed as a function of the temperature.

The waxes that may be used in the compositions according to the invention are chosen from waxes that are solid at ambient temperature of animal, vegetable, mineral or synthetic origin, and mixtures thereof.

As illustrations of waxes that are suitable for the invention, mention may be made especially of hydrocarbon-based waxes, for instance beeswax, lanolin wax, Chinese insect waxes, rice bran wax, carnauba wax, candelilla wax, ouricury wax, esparto wax, berry wax, shellac wax, Japan wax and sumac wax; montan wax, orange wax and lemon wax, refined sunflower wax sold under the name Sunflower Wax by Koster Keunen, microcrystalline waxes, paraffins and ozokerite; polyethylene waxes, the waxes obtained by the Fischer-Tropsch synthesis and waxy copolymers, and also esters thereof.

Mention may also be made of waxes obtained by catalytic hydrogenation of animal or vegetable oils containing linear or branched C₈-C₃₂ fatty chains. Mention may especially be made, among these waxes, of isomerized jojoba oil such as the trans-isomerized partially hydrogenated jojoba oil manufactured or sold by the company Desert Whale under the commercial reference Iso-Jojoba-50®, hydrogenated sunflower oil, hydrogenated castor oil, hydrogenated coconut oil, hydrogenated lanolin oil and bis(1,1,1-trimethylolpropane) tetrastearate sold under the name Hest 2T-4S® by the company Heterene.

Mention may also be made of silicone waxes (C₃₀₋₄₅ alkyl dimethicone) and fluoro waxes.

The waxes obtained by hydrogenation of castor oil esterified with cetyl alcohol, sold under the names Phytowax Castor 16L64® and 22L73® by the company Sophim, may also be used. Such waxes are described in application FR-A-2 792 190.
A wax that may be used is a C₂₀-C₄₀ alkyl (hydroxystearyloxy)stearate (the alkyl group containing from 20 to 40 carbon atoms), alone or as a mixture.

Such a wax is especially sold under the names Kester Wax K 82 P®, Hydroxypolyester K 82 P® and Kester Wax K 80 P® by the company Koster Keunen.

As microwaxes that may be used in the compositions according to the invention, mention may be made especially of carnauba microwaxes, such as the product sold under the name MicroCare 350® by the company Micro Powders, microwaxes of synthetic wax, such as the product sold under the name MicroEase 114S® by the company Micro Powders, microwaxes constituted of a mixture of carnauba wax and polyethylene wax, such as the products sold under the names Micro Care 300® and 310® by the company Micro Powders, microwaxes constituted of a mixture of carnauba wax and synthetic wax, such as the product sold under the name Micro Care 325® by the company Micro Powders, polyethylene microwaxes, such as the products sold under the names Micropoly 200®, 220®, 220L® and 250S® by the company Micro Powders, the commercial products Performalene 400 Polyethylene and Performalene 500-L Polyethylene from New Phase Technologies, Performalene 655 Polyethylene or paraffin waxes, for instance the wax having the INCl name Microcrystalline Wax and Synthetic Wax and sold under the trade name Microlease by the company Sochibo; polytetrafluoroethylene microwaxes such as those sold under the names Microslip 519® and 519 L® by the company Micro Powders.

The composition according to the invention will preferably comprise a content of wax(es) ranging from 3% to 20% by weight relative to the total weight of the composition, in particular from 5% to 15% and more particularly from 6% to 15%.

According to a particular form of the invention, in the context of anhydrous solid compositions in stick form, use will be made of polyethylene microwaxes in the form of crystallites with an aspect ratio at least equal to 2, and with a melting point ranging from 70°C to 110°C and preferably from 70°C to 100°C, in order to reduce or indeed even eliminate the presence of strata in the solid composition.

These crystallites in needle form and especially the dimensions thereof may be characterized visually according to the following method.

The wax is deposited on a microscope slide, which is placed on a hotplate. The slide and the wax are heated to a temperature generally at least 5°C higher than the melting point of the wax or of the mixture of waxes under consideration. At the end of melting, the liquid thus obtained and the microscope slide are allowed to cool in order to solidify. Observation of the crystallites is performed using a Leica DMLB100 optical microscope, with an objective lens selected as a function of the size of the objects to be viewed, and under polarized light. The dimensions of the crystallites are measured using image analysis software such as that sold by the company Microvision.

The crystallite polyethylene waxes in accordance with the invention preferably have an average length ranging from 5 to 10 µm. The term "average length" denotes the dimension given by the statistical particle size distribution at half the population, which is written as D50.

Use will be made more particularly of a mixture of Performalene 400 Polyethylene and Performalene 500-L Polyethylene waxes from New Phase Technologies.

### Pasty compounds

Within the meaning of the present invention, the term "pasty compound" is understood to mean a lipophilic fatty compound that undergoes a reversible solid/liquid change in state, which has in the solid state an anisotropic crystal organization, and that comprises, at a temperature of 23°C, a liquid fraction and a solid fraction.

The pasty compound is preferably chosen from synthetic compounds and compounds of vegetable origin. A pasty compound may be obtained by synthesis from starting materials of vegetable origin.

The pasty compound may be advantageously chosen from:
- lanolin and derivatives thereof,
- polymeric or non-polymeric silicone compounds,
- polymeric or non-polymeric fluoro compounds,
- vinyl polymers, especially:
- olefin homopolymers,
- olefin copolymers,
- hydrogenated diene homopolymers and copolymers,
- linear or branched oligomers, homopolymers or copolymers of alkyl (meth)acrylates preferably containing a C₈-C₃₀ alkyl group,
- oligomers, homopolymers and copolymers of vinyl esters containing C₈-C₃₀ alkyl groups,
- oligomers, homopolymers and copolymers of vinyl ethers containing C₈-C₃₀ alkyl groups,
- liposoluble polyethers resulting from the polyetherification between one or more C₂-C₁₀₀ and preferably C₂-C₅₀ diols,
- esters,
- mixtures thereof.

Among the esters, the following are especially preferred:
- esters of a glycerol oligomer, especially diglycerol esters, in particular condensates of adipic acid and of glycerol, for which some of the hydroxyl groups of the glycerols have reacted with a mixture of fatty acids such as stearic acid, capric acid, stearic acid and isostearic acid and 12-hydroxystearic acid, especially such as those sold under the brand name Softisan 649 by the company Sasol,
- the arachidyl propionate sold under the brand name Waxenol 801 by Alzo,
- phytosterol esters,
- fatty acid triglycerides and derivatives thereof,
- pentaerythritol esters,
- non-crosslinked polyesters resulting from polycondensation between a linear or branched C₄-C₅₀ dicarboxylic acid or polycarboxylic acid and a C₂-C₅₀ diol or polyol,
- aliphatic esters of an ester, resulting from the esterification of an aliphatic hydroxycarboxylic acid ester with an aliphatic carboxylic acid,
- polyesters resulting from the esterification, with a polycarboxylic acid, of an aliphatic hydroxycarboxylic acid ester, said ester comprising at least two hydroxyl groups, such as the products Risocast DA-H® and Risocast DA-L®,
- esters of a diol dimer and of a diacid dimer, where appropriate esterified on their free alcohol or acid functional group(s) by acid or alcohol radicals, such as Plandool-G,
- mixtures thereof.

Among the pasty compounds of vegetable origin a mixture of oxyethylenated (5 EO) oxypropylenated (5 PO) pentaerythritol and soybean sterols, sold under the reference Lanolide by the company Vevy, will preferably be chosen.

### Lipophilic gelling agents

### Mineral gelling agents

Mineral lipophilic gelling agents that may be mentioned include optionally modified clays, for instance hectorites modified with a C10-C22 ammonium chloride, for instance hectorite modified with distearyldimethylammonium chloride, for instance the product sold under the name Bentone 38V® by the company Elementis.

Mention may also be made of optionally hydrophobic-surface-treated fumed silica with a particle size of less than 1 µm. This is because it is possible to chemically modify the surface of the silica, by chemical reaction generating a reduced number of silanol groups present at the surface of the silica. It is possible in particular to substitute silanol groups with hydrophobic groups; a hydrophobic silica is then obtained. The hydrophobic groups may be trimethylsiloxyl groups, which are obtained especially by treating fumed silica in the presence of hexamethyldisilazane. Silicas thus treated are known as "silica silylate" according to the CTFA (8th Edition, 2000). They are sold, for example, under the references Aerosil R812® by the company Degussa, CAB-O-SIL TS-530® by the company Cabot, wherein the hydrophobic groups may be dimethylsilyloxyl or polydimethylsiloxane groups and those silica are obtained especially by treating fumed silica in the presence of polydimethylsiloxane or dimethyldichlorosilane. Silicas thus treated are known as "silica dimethyl silylate" according to the CTFA (8th Edition, 2000). They are sold, for example, under the references Aerosil R972® and Aerosil R974® by the company Degussa, and CAB-O-SIL TS-610® and CAB-O-SIL TS-720® by the company Cabot.

The hydrophobic fumed silica in particular has a particle size that may be nanometric to micrometric, for example ranging from about 5 to 200 nm.

### Organic gelling agents

The polymeric organic lipophilic gelling agents are, for example, partially or totally crosslinked elastomeric organopolysiloxanes of three-dimensional structure, for instance those sold under the names KSG6®, KSG16® and KSG18® by the company Shin-Etsu, Trefil E-505C® or Trefil E-506C® by the company Dow Corning, Gransil SR-CYC®, SR DMF10®, SR-DC556®, SR 5CYC gel®, SR DMF 10 gel® and SR DC 556 gel® by the company Grant Industries and SF 1204® and JK 113® by the company General Electric; ethyl cellulose, for instance the product sold under the name Ethocel® by the company Dow Chemical; galactomannans comprising from one to six and in particular from two to four hydroxyl groups per saccharide, substituted with a saturated or unsaturated alkyl chain, for instance guar gum alkylated with C₁ to C₆, and in particular C₁ to C₃, alkyl chains, and mixtures thereof. Block copolymers of "diblock", "triblock" or "radial" type, of the polystyrene/polyisoprene or polystyrene/polybutadiene type, such as the products sold under the name Luvitol HSB® by the company BASF, of the polystyrene/copoly(ethylenepropylene) type, such as the products sold under the name Kraton® by the company Shell Chemical Co., or of the polystyrene/copoly(ethylene-butylene) type, and mixtures of triblock and radial (star) copolymers in isododecane, such as those sold by the company Penreco under the name Versagel®, for instance the mixture of butylene/ethylene/styrene triblock copolymer and of ethylene/propylene/styrene star copolymer in isododecane (Versagel M 5960).

Lipophilic gelling agents that may also be mentioned include polymers with a weight-average molecular weight of less than 100 000, comprising a) a polymer backbone with hydrocarbon-based repeating units containing at least one heteroatom, and optionally b) at least one optionally functionalized pendent fatty chain and/or at least one optionally functionalized terminal fatty chain, containing from 6 to 120 carbon atoms and being linked to these hydrocarbon-based units, as described in patent applications WO-A-02/056 847 and WO-A-02/47619, in particular polyamide resins (especially comprising alkyl groups containing from 12 to 22 carbon atoms) such as those described in US-A-5 783 657.

Among the lipophilic gelling agents that may be used in the compositions according to the invention, mention may also be made of fatty acid esters of dextrin, such as dextrin palmitates, especially the products sold under the names Rheopearl TL® or Rheopearl KL® by the company Chiba Flour.

It is also possible to use silicone polyamides of the polyorganosiloxane type such as those described in documents US-A-5 874 069, US-A-5 919 441, US-A-6 051 216 and US-A-5 981 680.

These silicone polymers may belong to the following two families:
- polyorganosiloxanes comprising at least two groups capable of establishing hydrogen interactions, these two groups being located in the chain of the polymer, and/or
- polyorganosiloxanes comprising at least two groups capable of establishing hydrogen interactions, these two groups being located on grafts or branches.

### ORGANIC POWDER

According to one particular form of the invention, the compositions according to the invention will also contain an organic powder.

In the present application, the term "organic powder" means any solid that is insoluble in the medium at ambient temperature (25°C).

As organic powders that may be used in the composition of the invention, examples that may be mentioned include polyamide particles and especially those sold under the Orgasol names by the company Atochem; nylon-6,6 fibres, especially the polyamide fibres sold by Etablissements P Bonte under the name Polyamide 0.9 Dtex 0.3 mm (INCl name: Nylon-6,6 or Polyamide-6,6) with a mean diameter of 6 µm, a weight of about 0.9 dtex and a length ranging from 0.3 mm to 1.5 mm; polyethylene powders; microspheres based on acrylic copolymers, such as those made of ethylene glycol dimethacrylate/lauryl methacrylate copolymer, sold by the company Dow Corning under the name Polytrap; polymethyl methacrylate microspheres, sold under the name Microsphere M-100 by the company Matsumoto or under the name Covabead LH85 by the company Wackherr; hollow polymethyl methacrylate microspheres (particle size: 6.5-10.5 µm) sold under the name Ganzpearl GMP 0800 by Ganz Chemical; methyl methacrylate/ethylene glycol dimethacrylate copolymer microbeads (size: 6.5-10.5 µm) sold under the name Ganzpearl GMP 0820 by Ganz Chemical or Microsponge 5640 by the company Amcol Health & Beauty Solutions; ethylene-acrylate copolymer powders, such as those sold under the name Flobeads by the company Sumitomo Seika Chemicals; expanded powders such as hollow microspheres and especially microspheres formed from a terpolymer of vinylidene chloride, acrylonitrile and methacrylate and sold under the name Expancel by the company Kemanord Plast under the references 551 DE 12 (particle size of about 12 µm and density of 40 kg/m³), 551 DE 20 (particle size of about 30 µm and density of 65 kg/m³), 551 DE 50 (particle size of about 40 µm), or the microspheres sold under the name Micropearl F 80 ED by the company Matsumoto; powders of natural organic materials such as starch powders, especially of crosslinked or non-crosslinked corn, wheat or rice starch, such as the powders of starch crosslinked with octenylsuccinic anhydride, sold under the name Dry-Flo by the company National Starch; silicone resin microbeads such as those sold under the name Tospearl by the company Toshiba Silicone, especially Tospearl 240; amino acid powders such as the lauroyl lysine powder sold under the name Amihope LL-11 by the company Ajinomoto; particles of wax microdispersion, which preferably have mean sizes of less than 1 µm and especially ranging from 0.02 µm to 1 µm, and which are essentially constituted of a wax or a mixture of waxes, such as the products sold under the name Aquacer by the company Byk Cera, and especially: Aquacer 520 (mixture of synthetic and natural waxes), Aquacer 514 or 513 (polyethylene wax), Aquacer 511 (polymeric wax), or such as the products sold under the name Jonwax 120 by the company Johnson Polymer (mixture of polyethylene wax and paraffin wax) and under the name Ceraflour 961 by the company Byk Cera (micronized modified polyethylene wax); and mixtures thereof.

### ADDITIVES

The cosmetic compositions according to the invention may also comprise cosmetic adjuvants chosen from softeners, antioxidants, opacifiers, stabilizers, moisturizers, vitamins, bactericides, preserving agents, polymers, fragrances, thickeners or suspension agents, propellants or any other ingredient usually used in cosmetics for this type of application.

Needless to say, a person skilled in the art will take care to select this or these optional additional compounds such that the advantageous properties intrinsically associated with the cosmetic composition in accordance with the invention are not, or are not substantially, adversely affected by the envisaged addition(s).

### THICKENERS AND SUSPENSION AGENTS

The thickeners may be chosen from carboxyvinyl polymers, such as Carbopols (Carbomers) and the Pemulens (acrylate/C10-C30 alkyl acrylate copolymer); polyacrylamides, for instance the crosslinked copolymers sold under the names Sepigel 305 (CTFA name: polyacrylamide/C¹³⁻¹⁴ isoparaffin/Laureth 7) or Simulgel 600 (CTFA name: acrylamide/sodium acryloyldimethyltaurate copolymer/isohexadecane/polysorbate 80) by the company SEPPIC; 2-acrylamido-2-methylpropanesulfonic acid polymers and copolymers, optionally crosslinked and/or neutralized, for instance poly(2-acrylamido-2-methylpropanesulfonic acid) sold by the company Hoechst under the trade name Hostacerin AMPS (CTFA name: ammonium polyacryloyldimethyltaurate) or Simulgel 800 sold by the company SEPPIC (CTFA name: sodium polyacryloyldimethyltaurate/polysorbate 80/sorbitan oleate); copolymers of 2-acrylamido-2-methylpropanesulfonic acid and of hydroxyethyl acrylate, for instance Simulgel NS and Sepinov EMT 10 sold by the company SEPPIC; cellulose derivatives such as hydroxyethyl cellulose or cetyl hydroxyethyl cellulose; polysaccharides and especially gums such as xanthan gum and hydroxypropyl guar gums; silicas, for instance Bentone Gel MIO sold by the company NL Industries or Veegum Ultra sold by the company Polyplastic.

The thickeners may also be cationic, for instance Polyquaternium-37 sold under the name Salcare SC95 (Polyquaternium-37 (and) Mineral Oil (and) PPG-1 Trideceth-6) or Salcare SC96 (Polyquaternium-37 (and) Propylene Glycol Dicaprylate/Dicaprate (and) PPG-1 Trideceth-6) or other crosslinked cationic polymers, for instance those of the CTFA name Ethyl Acrylate/Dimethylaminoethyl Methacrylate Cationic Copolymer In Emulsion.

### SUSPENSION AGENTS

In order to improve the homogeneity of the product, it is also possible to use one or more suspension agents preferably chosen from hydrophobic modified montmorillonite clays such as hydrophobic modified bentonites or hectorites. Examples that may be mentioned include the product Stearalkonium Bentonite (CTFA name) (reaction product of bentonite and the quaternary ammonium stearalkonium chloride) such as the commercial product sold under the name Tixogel MP 250 by the company Sud Chemie Rheologicals, United Catalysts Inc. or the product Disteardimonium Hectorite (CTFA name) (reaction product of hectorite and distearyldimonium chloride) sold under the name Bentone 38 or Bentone Gel by the company Elementis Specialities.

Other suspension agents may be used, in the present case in hydrophilic (aqueous and/or ethanolic) media. They may be cellulose, xanthan, guar, starch, locust bean or agar agar derivatives.

The suspension agents are preferably present in amounts ranging from 0.1% to 5% by weight and more preferentially from 0.2% to 2% by weight relative to the total weight of the composition.

The amounts of these various constituents that may be present in the cosmetic composition according to the invention are those conventionally used in compositions for treating perspiration.

### AEROSOLS

The compositions according to the invention may also be pressurized and may be packaged in an aerosol device formed by:
(A) a container comprising an antiperspirant composition as defined previously,
(B) at least one propellant and a means for dispensing said aerosol composition.

The propellants generally used in products of this type and that are well known to those skilled in the art are, for instance, dimethyl ether (DME); volatile hydrocarbons such as n-butane, propane, isobutane and mixtures thereof, optionally with at least one chlorinated and/or fluorinated hydrocarbon; among the latter, mention may be made of the compounds sold by the company DuPont de Nemours under the names Fréon® and Dymel®, and in particular monofluorotrichloromethane, difluorodichloromethane, tetrafluorodichloroethane and 1,1-difluoroethane sold especially under the trade name Dymel 152 A by the company DuPont. Carbon dioxide, nitrous oxide, nitrogen or compressed air may also be used as propellant.

The compositions containing perlite particles as defined previously and the propellant(s) may be in the same compartment or in different compartments in the aerosol container. According to the invention, the concentration of propellant generally varies from 5% to 95% by weight of pressurized composition, and more preferentially from 50% to 85% by weight relative to the total weight of the pressurized composition.

The dispensing means, which forms a part of the aerosol device, is generally formed by a dispensing valve controlled by a dispensing head, which itself comprises a nozzle via which the aerosol composition is vaporized. The container containing the pressurized composition may be opaque or transparent. It may be made of glass, polymer or metal, optionally coated with a protective varnish coat.

The expressions "between ... and ..." and "ranging from ... to ..." should be understood as meaning limits included, unless otherwise specified.

The examples that follow illustrate the present invention without limiting the scope thereof.

### Examples 1a, 1b and 2: Roll-on (O/W emulsion)

Compositions 1 a, 1b and 2 were prepared according to the following procedure.

| **Phase** | **Ingredients** | **Example 1a or 1b (invention)** | **Example 2 (outside the invention)** |
|---|---|---|---|
| A | Demineralized water | qs 100 | qs 100 |
| B | Steareth-100/PEG-136/HDI Copolymer RHEOLATE FX 1100 (ELEMENTIS) | 1 | 1 |
| C | Aluminium chlorohydrate (50% aqueous solution) (SUMMITREHEIS) | 10 | 10 |
| D | n-Octanoylsalicylic acid MEXORYL SAB (CHIMEX) | 0.2 | 0.2 |
| E | Dimethicone BELSIL DM 350 (WACKER) | 7 | 7 |
| F | Preservative | 0.075 | 0.075 |
| | Tetrasodium Glutamate Diacetate Dissolvine GL-47-S (AKZO NOBEL) | 0.15% active material | - |

### Preparation method

### Common steps of the process

1- Phase B is sprinkled into phase A with stirring using a Rayneri machine (30 min) while heating to 80°C.
2- When the mixture AB is homogeneous, phases C and F are added using a Turax machine (speed 1) at 80°C.
3- Phase D is heated to 80°C on a hotplate with magnetic stirring.
4- When the mixture ABCF and phase D are at 80°C, phase D is emulsified in the mixture ABCF with stirring using a Turax machine (speed 2) for 20 min. The heating is stopped.
5- The mixture is stirred using a Rayneri machine and phase E is added, then it is left to cool to 25°C.
6- At 25°C, the formula is stopped.

### Example 1a

In step 2, when the mixture AB is homogeneous, phase C is added in the presence of the amino acid-N,N-diacetic acid salt using a Turax machine (speed 1) at 80°C.

### Example 1b

In step 5, after adding phase E, the amino acid-N,N-diacetic acid salt is added at 70°C and then the mixture is left to cool to 25°C.

The stability of the compositions during storage is evaluated over two months at 37°C and 45°C. The appearance of each composition, and more particularly the colour, is observed at T0 and after two months. The colour of the formulae at T0 is white.

| **Composition** | **Appearance after two months at 37°C and 45°C** |
|---|---|
| **Example 1a** | Absence of colouration |
| **Example 1b** | Very slightly pinkish white |
| **Example 2 (outside the invention)** | Pale pink at 37°C and 45°C |

It was observed that the compositions 1 a and 1 b according to the invention that contain the amino acid-N,N-diacetic acid salt are more stable on storage than the composition 2 that does not contain this sequestrant.

It was observed with composition 1 a that the preparation process in which the sequestrant is added in the presence of the aluminium salt before being brought into contact with the lipophilic salicylic derivative and before the formation of the emulsion improves the storage stability of the final composition obtained.

### Examples 3a, 3b and 4: Roll-on (O/W emulsion)

Compositions 3a, 3b and 4 were prepared according to the following procedure.

| **Phase** | **Ingredients** | **Example 3a or 3b (invention)** | **Example 4 (outside the invention)** |
|---|---|---|---|
| A | Demineralized water | qs 100 | qs 100 |
| | Aluminium chlorohydrate (50% aqueous solution) (SUMMITREHEIS) | 10% (5% active material) | 10% (5% active material) |
| B | PPG-15 stearyl ether ARLAMOL PS15E-LQ-(RB) (CRODA) | 3 | 3 |
| | n-Octanoylsalicylic acid MEXORYL SAB (CHIMEX) | 0.2 | 0.2 |
| | Dimethicone BELSIL DM 350 (WACKER) | 0.5 | 0.5 |
| | Ceteareth-33 SIMULSOL CS FLAKES (SEPPIC) | 1.25 | 1.25 |
| | Cetearyl alcohol LAN ETTE D (COGNIS) | 2.5 | 2.5 |
| C | Preservative | 0.075 | 0.075 |
| | Tetrasodium Glutamate Diacetate Dissolvine GL-47-S (AKZO NOBEL) | 0.15% active material | - |

### Preparation method

### Common steps of the process

1- Phase A is heated to 80°C using a Turax machine (speed 1).
2- Phase B is melted at 80°C on a hotplate with magnetic stirring.
3- Phase B is introduced into phase A using a Turax machine (speed 2) at 80°C over 15 minutes.
4- The mixture is stirred using a Rayneri machine.
5- Phase C is added to the mixture and it is left to cool to 25°C.
6- At 25°C, the formula is stopped.

### Example 3a

After step 1 of heating phase A, the amino acid-N,N-diacetic acid salt is introduced therein.

### Example 3b

In step 5, after adding phase C to the mixture, the amino acid-N,N-diacid salt is added and then the mixture is left to cool to 25°C.

The stability of the compositions during storage is evaluated over two months at 37°C and 45°C. The appearance of each composition, and more particularly the colour, is observed at T0 and after two months. The colour of the formulae at T0 is white.

| **Composition** | **Appearance after two months at 37°C and 45°C** |
|---|---|
| **Example 3a** | Absence of colouration |
| **Example 3b** | Very slightly pinkish white |
| **Example 4 (outside the invention)** | Pale pink at 37°C and 45°C |

It was observed that the compositions 3a and 3b according to the invention that contain the amino acid-N,N-diacetic acid salt are more stable on storage than the composition 4 that does not contain this sequestrant.

It was observed with composition 3a that the preparation process in which the sequestrant is added in the presence of the aluminium salt before being brought into contact with the lipophilic salicylic derivative and before the formation of the emulsion improves the storage stability of the final composition obtained.

## Claims

1. Cosmetic composition comprising, in a cosmetically acceptable medium, at least:
a) a lipophilic salicylic acid derivative of general formula (I) below or a salt thereof derived from a mineral or organic base: 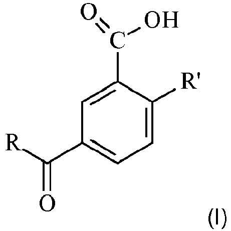 in which:
- the R radical denotes a linear, branched or cyclic, saturated aliphatic chain containing from 2 to 22 carbon atoms; an unsaturated chain containing from 2 to 22 carbon atoms and comprising one or more double bonds that may be conjugated; an aromatic ring bonded to the carbonyl radical directly or via saturated or unsaturated aliphatic chains containing from 2 to 7 carbon atoms; it being possible for said groups to be substituted with one or more substituents, which may be identical or different, chosen from (a) halogen atoms, (b) a trifluoromethyl group, (c) hydroxyl groups in free form or esterified with an acid containing from 1 to 6 carbon atoms, or (d) a carboxyl function in free form or esterified with a lower alcohol containing from 1 to 6 carbon atoms;
- R' is a hydroxyl group or an ester group of formula: 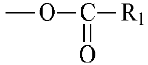 in which R₁ denotes a linear or branched, saturated or unsaturated aliphatic chain containing from 1 to 18 carbon atoms;
b) at least one antiperspirant aluminium salt or complex; and
c) at least one sequestrant chosen from amino acid-N,N-diacetic acid salts.

2. Composition according to Claim 1, where the compound of formula (I) is chosen from those for which the R radical denotes a linear, branched or cyclic, saturated aliphatic chain containing from 3 to 11 carbon atoms; an unsaturated chain containing from 3 to 17 carbon atoms and comprising one or more conjugated or unconjugated double bonds; it being possible for said hydrocarbon-based chains to be substituted with one or more substituents, which may be identical or different, chosen from (a) halogen atoms, (b) a trifluoromethyl group, (c) hydroxyl groups in free form or esterified with an acid containing from 1 to 6 carbon atoms, or (d) a carboxyl function in free form or esterified with a lower alcohol containing from 1 to 6 carbon atoms;
- R' is a hydroxyl group or an ester group of formula: 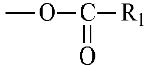 in which R₁ denotes a radical -O-(C=O)-(CH₂)ₙ-CH₃ where n is a number ranging from 0 to 14;
- and also salts thereof obtained by salification with a mineral or organic base.

3. Composition according to Claim 1 or 2, where the compound of formula (I) is chosen from those for which the R radical is a C₃-C₁₁ alkyl group and R' denotes hydroxyl.

4. Composition according to any one of Claims 1 to 3, where the compound of formula (I) is chosen from those for which R represents a chain derived from linoleic, linolenic or oleic acid.

5. Composition according to any one of Claims 1 to 4, where the compound of formula (I) is chosen from those for which the R radical denotes a C₃-C₁₁ alkyl group bearing a carboxyl function in free form or esterified with a lower alcohol containing from 1 to 6 carbon atoms and R' denotes hydroxyl.

6. Composition according to any one of the preceding claims, where the compound of formula (I) is chosen from 5-n-octanoylsalicylic acid (or capryloyl salicylic acid); 5-n-decanoylsalicylic acid; 5-n-dodecanoylsalicylic acid; 5-n-heptyloxysalicylic acid and the salts thereof derived from a mineral or organic base.

7. Composition according to Claim 6, where the compound of formula (I) is 5-n-octanoylsalicylic acid (or capryloyl salicylic acid) and the salts thereof derived from a mineral or organic base.

8. Composition according to any one of Claims 1 to 7, where the antiperspirant aluminium salt or complex is chosen from aluminium halohydrates; aluminium zirconium halohydrates; and complexes of zirconium hydroxychloride and of aluminium hydroxychloride with an amino acid.

9. Composition according to any one of the preceding claims, where the antiperspirant aluminium salt or complex is aluminium chlorohydrate in activated or non-activated form.

10. Composition according to any one of the preceding claims, where, in the chemical structure of the amino acid-N,N-diacetic acid salt, the amino acid-N,N-diacetic acid is chosen from:
- glutamic acid-N,N-diacetic acid of the following structure: 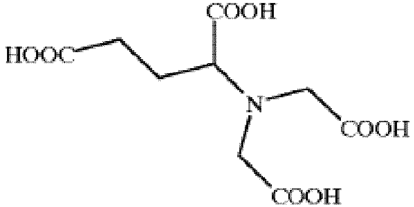
- α-alanine-N,N-diacetic acid of the following structure: 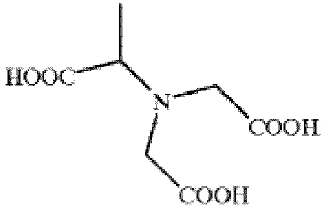
- β-alanine-N,N-diacetic acid of the following structure: 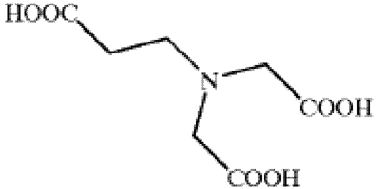
- aspartic acid-N,N-diacetic acid of the following structure: 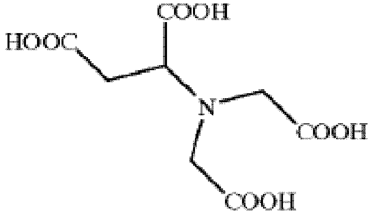
- glycine-N,N-diacetic acid of the following structure: 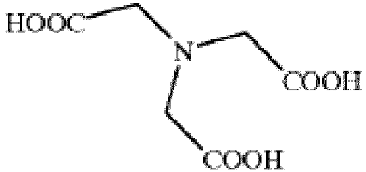
- serine-N,N-diacetic acid of the following structure: 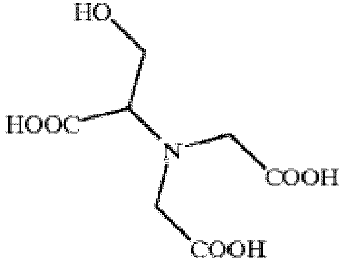 and more particularly glutamic acid-N,N-diacetic acid.

11. Composition according to any one of the preceding claims, where, in the amino acid-N,N-diacetic acid salt, all the carboxylic functions are salified with a mineral base and/or an organic base.

12. Composition according to Claim 11, where the mineral base is chosen from alkali metal hydroxides, alkaline-earth metal hydroxides or aqueous ammonia and the organic base is chosen from primary, secondary or tertiary amines, in particular alkanolamines.

13. Composition according to Claim 12, where, in the amino acid-N,N-diacetic acid salt, all the carboxylic functions are in sodium salt form.

14. Composition according to Claim 13, where the amino acid-N,N-diacetic acid salt is glutamic acid-N,N-diacetic acid tetrasodium salt.

15. Composition according to any one of the preceding claims, **characterized in that** it is in the form of an emulsion comprising at least one aqueous phase and at least one fatty phase, in particular in the form of an oil-in-water emulsion.

16. Process for preparing a composition according to Claim 15, **characterized in that** the amino acid-N,N-diacetic acid salt is introduced in the presence of the aluminium salt(s) or complex(es) before mixing with the amino acid-N,N-diacetic acid salt and before emulsification of the aqueous phase and of the fatty phase.

17. Cosmetic process for treating human perspiration and optionally body odours, which consists in applying an effective amount of a composition as defined according to any one of Claims 1 to 15 to the surface of the skin.

## Patentansprüche

1. Kosmetikzusammensetzung, die in einen kosmetisch unbedenklichen Medium mindestens Folgendes umfasst:
a) ein lipophiles Salizylsäurederivat der allgemeinen Formel (I) unten oder ein Salz davon, das sich von einer anorganischen oder organischen Base ableitet: 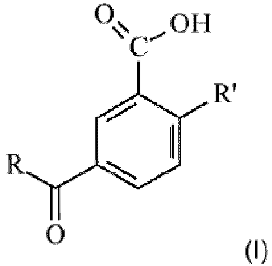 in der:
- der Rest R eine geradkettige, verzweigte oder cyclische gesättigte aliphatische Kette mit 2 bis 22 Kohlenstoffatomen; eine ungesättigte Kette mit 2 bis 22 Kohlenstoffatomen, die eine oder mehrere Doppelbindungen, die konjugiert sein können, umfasst; einen aromatischen Ring, der direkt oder über gesättigte oder ungesättigte aliphatische Ketten mit 2 bis 7 Kohlenstoffatomen an den Carbonylrest gebunden ist; wobei die genannten Gruppen mit einem oder mehreren Substituenten substituiert sein können, die gleich oder verschieden sein können und aus (a) Halogenatomen, (b) einer Trifluormethylgruppe, (c) Hydroxylgruppen, die in freier Form vorliegen oder mit einer Säure mit 1 bis 6 Kohlenstoffatomen verestert sind, oder (d) einer Carboxylfunktion, die in freier Form vorliegt oder mit einem Niederalkohol mit 1 bis 6 Kohlenstoffatomen verestert ist, ausgewählt sind;
- R' eine Hydroxylgruppe oder eine Estergruppe der Formel: 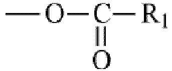 bedeutet,
in der R₁ eine geradkettige oder verzweigte gesättigte oder ungesättigte aliphatische Kette mit 1 bis 18 Kohlenstoffatomen bedeutet;
b) mindestens ein Antiperspirans-Aluminiumsalz oder einen Komplex davon; und
c) mindestens einen Chelatbildner aus der Reihe der Aminosäure-N,N-diessigsäuresalze.

2. Zusammensetzung nach Anspruch 1, wobei die Verbindung der Formel (I) aus denjenigen ausgewählt ist, bei denen der Rest R eine geradkettige, verzweigte oder cyclische gesättigte aliphatische Kette mit 3 bis 11 Kohlenstoffatomen; eine ungesättigte Kette mit 3 bis 17 Kohlenstoffatomen, die eine oder mehrere konjugierte oder nicht konjugierte Doppelbindungen umfasst, bedeutet; wobei die genannten auf Kohlenstoff basierenden Ketten mit einem oder mehreren Substituenten substituiert sein können, die gleich oder verschieden sein können und aus (a) Halogenatomen, (b) einer Trifluormethylgruppe, (c) Hydroxylgruppen, die in freier Form vorliegen oder mit einer Säure mit 1 bis 6 Kohlenstoffatomen verestert sind, oder (d) einer Carboxylfunktion, die in freier Form vorliegt oder mit einem Niederalkohol mit 1 bis 6 Kohlenstoffatomen verestert ist, ausgewählt sind;
- R' eine Hydroxylgruppe oder eine Estergruppe der Formel: 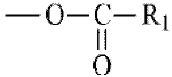 bedeutet,
in der R₁ einen Rest -O-(C=O)-(CH₂)ₙ-CH₃ bedeutet, wobei n eine Zahl von 0 bis 14 ist;
- sowie Salzen davon, die durch Salzbildung mit einer anorganischen oder organischen Base erhalten werden.

3. Zusammensetzung nach Anspruch 1 oder 2, wobei die Verbindung der Formel (I) aus denjenigen ausgewählt ist, bei denen der Rest R eine C₃-C₁₁-Alkylgruppe ist und R' Hydroxyl bedeutet.

4. Zusammensetzung nach einem der Ansprüche 1 bis 3, wobei die Verbindung der Formel (I) aus denjenigen ausgewählt ist, bei denen R eine Kette bedeutet, die sich von Linolsäure, Linolensäure oder Ölsäure ableitet.

5. Zusammensetzung nach einem der Ansprüche 1 bis 4, wobei die Verbindung der Formel (I) aus denjenigen ausgewählt ist, bei denen der Rest R eine C₃-C₁₁-Alkylgruppe bedeutet, die eine Carboxylfunktion trägt, die in freier Form vorliegt oder mit einem Niederalkohol mit 1 bis 6 Kohlenstoffatomen verestert ist, und R' Hydroxyl bedeutet.

6. Zusammensetzung nach einem der vorhergehenden Ansprüche, wobei die Verbindung der Formel (I) aus 5-n-Octanoylsalicylsäure (oder Capryloylsalicylsäure), 5-n-Decanoylsalicylsäure, 5-n-Dodecanoylsalicylsäure, 5-n-Heptyloxysalicylsäure und ihren Salzen, die sich von einer anorganischen oder organischen Base ableiten, ausgewählt ist.

7. Zusammensetzung nach Anspruch 6, wobei es sich bei der Verbindung der Formel (I) um 5-n-Octanoylsalicylsäure (oder Capryloylsalicylsäure) und den Salzen davon, die sich von einer anorganischen oder organischen Base ableiten, handelt.

8. Zusammensetzung nach einem der Ansprüche 1 bis 7, wobei das Antiperspirans-Aluminiumsalz oder der Komplex davon aus Aluminiumhalogenhydraten, Aluminiumzirconiumhalogenhydraten und Komplexen von Zirconiumhydroxychlorid und von Aluminiumhydroxychlorid mit einer Aminosäure ausgewählt ist.

9. Zusammensetzung nach einem der vorhergehenden Ansprüche, wobei es sich bei dem Antiperspirans-Aluminiumsalz oder dem Komplex davon um Aluminiumhydrochlorid in aktivierter oder nicht aktivierter Form handelt.

10. Zusammensetzung nach einem der vorhergehenden Ansprüche, wobei die Aminosäure-N,N-diessigsäure in der chemischen Struktur des Aminosäure-N,N-diessigsäure-salzes aus den Folgenden ausgewählt ist:
- Glutaminsäure-N,N-diessigsäure der folgenden Struktur: 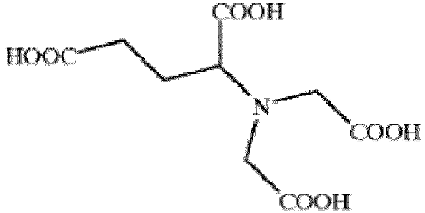
- α-Alanin-N,N-diessigsäure der folgenden Struktur: 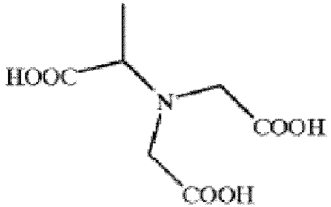
- β-Alanin-N,N-diessigsäure der folgenden Struktur: 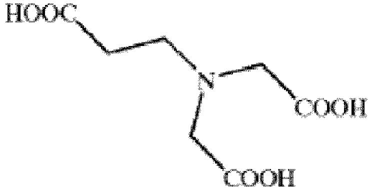
- Asparaginsäure-N,N-diessigsäure der folgenden Struktur: 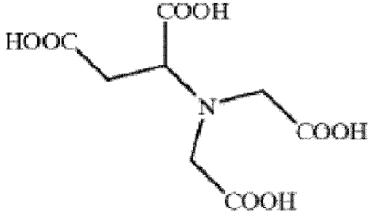
- Glycin-N,N-diessigsäure der folgenden Struktur: 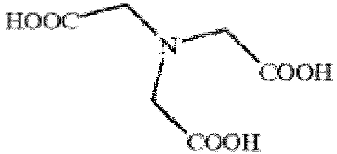
- Serin-N,N-diessigsäure der folgenden Struktur: 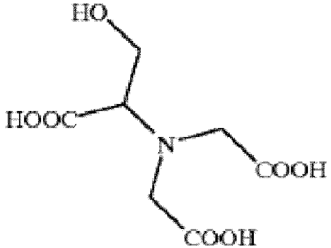 und insbesondere Glutaminsäure-N,N-diessigsäure.

11. Zusammensetzung nach einem der vorhergehenden Ansprüche, wobei alle Carbonsäurefunktionen in dem Aminosäure-N,N-diessigsäuresalz einer Salzbildung mit einer anorganischen Base und/oder einer organischen Base unterworfen worden sind.

12. Zusammensetzung nach Anspruch 11, wobei die anorganische Base aus Alkalimetallhydroxiden, Erdalkalimetallhydroxiden oder wässrigem Ammoniak ausgewählt ist und die organische Base aus primären, sekundären oder tertiären Aminen, insbesondere Alkanolaminen, ausgewählt ist.

13. Zusammensetzung nach Anspruch 12, wobei alle Carbonsäurefunktionen in dem Aminosäure-N,N-diessigsäuresalz in Form des Natriumsalzes vorliegen.

14. Zusammensetzung nach Anspruch 13, wobei es sich bei dem Aminosäure-N,N-diessigsäuresalz um Glutaminsäure-N,N-diessigsäuretetranatriumsalz handelt.

15. Zusammensetzung nach einem der vorhergehenden Ansprüche, **dadurch gekennzeichnet, dass** sie in Form einer Emulsion, die mindestens eine wässrige Phase und mindestens eine Fettphase umfasst, insbesondere in Form einer Öl-in-Wasser-Emulsion, vorliegt.

16. Verfahren zur Herstellung einer Zusammensetzung nach Anspruch 15, **dadurch gekennzeichnet, dass** das Aminosäure-N,N-diessigsäuresalz in Gegenwart des Aluminiumsalzes bzw. der Aluminiumsalze oder des Komplexes bzw. der Komplexe davon eingeführt wird, bevor man mit dem Aminosäure-N,N-diessigsäuresalz vermischt und bevor die wässrige Phase und die Fettphase emulgiert werden.

17. Kosmetisches Verfahren zum Behandeln von Schweißbildung des Menschen und gegebenenfalls von Körpergerüchen, das darin besteht, dass man eine wirksame Menge einer Zusammensetzung wie in einem der Ansprüche 1 bis 15 definiert auf die Oberfläche der Haut aufträgt.

## Revendications

1. Composition cosmétique comprenant dans un milieu cosmétiquement acceptable au moins :
a) un dérivé lipophile d'acide salicylique de formule générale (I) suivante ou l'un de ses sels issus d'une base minérale ou organique 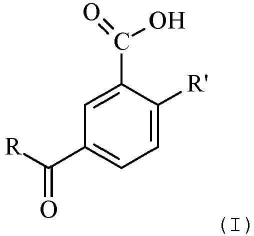 dans laquelle :
- le radical R désigne une chaîne aliphatique ayant de 2 à 22 atomes de carbone, saturée, linéaire, ramifiée ou cyclique ; une chaîne insaturée ayant de 2 à 22 atomes de carbone contenant une ou plusieurs doubles liaisons pouvant être conjuguées ; un noyau aromatique lié au radical carbonyle directement ou par l'intermédiaire de chaînes aliphatiques saturées ou insaturées ayant de 2 à 7 atomes de carbone; lesdits groupes pouvant être substitués par un ou plusieurs substituants, identiques ou différents, choisis parmi (a) les atomes d'halogène, (b) un groupe trifluorométhyle, (c) des groupes hydroxyle sous forme libre ou estérifiée par un acide ayant de 1 à 6 atomes de carbone, ou (d) une fonction carboxyle sous forme libre ou estérifiée par un alcool inférieur ayant de 1 à 6 atomes de carbone ;
- R' est un groupe hydroxyle ou groupe ester de formule : 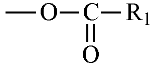 dans laquelle R₁ désigne une chaîne aliphatique, saturée ou insaturée, linéaire ou ramifiée contenant de 1 à 18 atomes de carbone ;
b) au moins un sel ou complexe d'aluminium anti-transpirant et
c) au moins un agent séquestrant choisi parmi les sels d'acide aminé-acide N,N-diacétique.

2. Composition selon la revendication 1, où le composé de formule (I) est choisi parmi ceux pour lesquels le radical R désigne une chaîne aliphatique saturée, linéaire, ramifiée ou cyclique contenant de 3 à 11 atomes de carbone ; une chaîne insaturée contenant de 3 à 17 atomes de carbone et comprenant une ou plusieurs doubles liaisons conjuguées ou non ; lesdites chaînes hydrocarbonées pouvant être substituées par un ou plusieurs substituants, identiques ou différents, choisis parmi (a) les atomes d'halogène, (b) un groupe trifluorométhyle, (c) des groupes hydroxyle sous forme libre ou estérifiée par un acide ayant de 1 à 6 atomes de carbone, ou (d) une fonction carboxyle sous forme libre ou estérifiée par un alcool inférieur ayant de 1 à 6 atomes de carbone ;
- R' est un groupe hydroxyle ou groupe ester de formule : 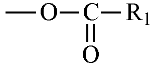 dans laquelle R₁ désigne un radical -O-(C=O)-(CH₂)ₙ-CH₃ où n est un nombre allant de 0 à 14 ;
- ainsi que leurs sels obtenus par salification par une base minérale ou organique.

3. Composition selon la revendication 1 ou 2, où le composé de formule (I) est choisi parmi ceux pour lesquels le radical R est un groupement alkyle en C₃-C₁₁ et R' désigne hydroxyle.

4. Composition selon l'une quelconque des revendications 1 à 3, où le composé de formule (I) est choisi parmi ceux pour lesquels R représente une chaîne dérivée de l'acide linoléique, linolénique ou oléique.

5. Composition selon l'une quelconque des revendications 1 à 4, où le composé de formule (I) est choisi parmi ceux pour lesquels le radical R désigne un groupement alkyle en C₃-C₁₁ portant une fonction carboxyle sous forme libre ou estérifiée par un alcool inférieur ayant de 1 à 6 atomes de carbone et R' désigne hydroxyle.

6. Composition selon l'une quelconque des revendications précédentes, où le composé de formule (I) est choisi parmi l'acide n-octanoyl-5-salicylique (ou acide capryloyl salicylique) ; l'acide n-décanoyl-5-salicylique ; l'acide n-dodécanoyl-5-salicylique ; l'acide n-heptyloxy-5-salicylique et leurs sels issus d'une base minérale ou organique.

7. Composition selon la revendication 6, où le composé de formule (I) est l'acide n-octanoyl-5-salicylique (ou acide capryloyl salicylique) et ses sels issus d'une base minérale ou organique.

8. Composition selon l'une quelconque des revendications 1 à 7, où le sel ou complexe d'aluminium anti-transpirant est choisi parmi les halogénohydrates d'aluminium ; les halogénohydrates d'aluminium et de zirconium ; les complexes d'hydroxychlorure de zirconium et d'hydroxychlorure d'aluminium avec un acide aminé.

9. Composition selon l'une quelconque des revendications précédentes, où le sel ou complexe d'aluminium anti-transpirant est le chlorhydrate d'aluminium sous forme activée ou non activée.

10. Composition selon l'une quelconque des revendications précédentes, où dans la structure chimique du sel d'acide aminé-acide N,N-diacétique, l'acide aminé-acide N,N-diacétique est choisi parmi
- l'acide glutamique-acide N,N-diacétique de structure suivante : 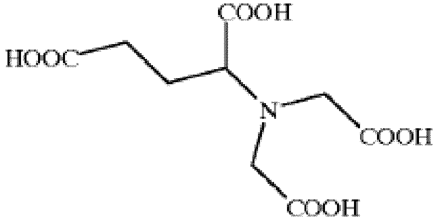
- l'α-alanine-acide N,N-diacétique de structure suivante : 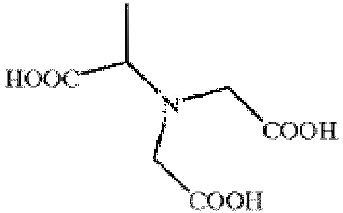
- la β-alanine-acide N,N-diacétique de structure suivante : 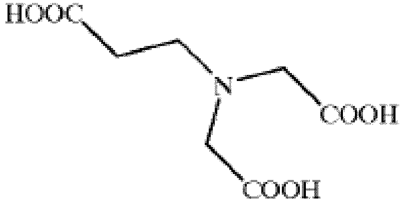
- l'acide aspartique-acide N,N-diacétique de structure suivante : 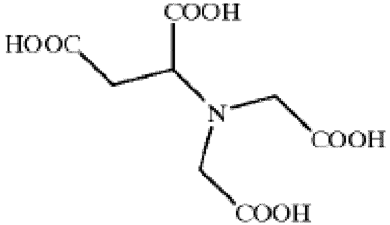
- la glycine-acide N,N-diacétique de structure suivante : 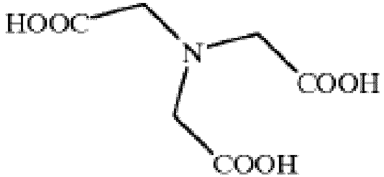
- la sérine-acide N,N-diacétique de structure suivante : 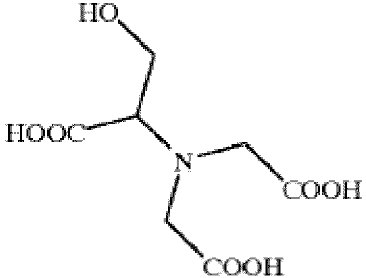 et plus particulièrement l'acide glutamique N,N-diacide acétique.

11. Composition selon l'une quelconque des revendications précédentes, où dans le sel d'acide aminé N,N-diacide acétique, toutes les fonctions carboxyliques sont salifiées par une base minérale et/ou une base organique.

12. Composition selon la revendication 11, où la base minérale est choisie parmi les hydroxydes de métal alcalin, les hydroxydes de métal alcalino-terreux ou l'ammoniaque et la base organique est choisie parmi les amines primaires, secondaires ou tertiaires notamment les alcanolamines.

13. Composition selon la revendication 12, où dans le sel d'acide aminé N,N-diacide acétique, toutes les fonctions carboxyliques sont sous forme de sel de sodium.

14. Composition selon la revendication 13, où le sel d'acide aminé N,N-diacide acétique est le sel tétrasodique de l'acide glutamique-acide N,N-diacétique.

15. Composition selon l'une quelconque des revendications précédentes, **caractérisée par le fait qu'**elle est sous forme d'émulsion comprenant au moins une phase aqueuse et au moins une phase grasse, en particulier sous forme d'émulsion huile-dans-eau.

16. Procédé de préparation d'une composition selon la revendication 15, **caractérisé en ce que** le sel d'acide aminé N,N-diacide acétique est introduit en présence du ou des sels ou complexes d'aluminium avant mélange avec le sel d'acide aminé N, N-diacide acétique et avant émulsification de la phase aqueuse et de la phase grasse.

17. Procédé cosmétique pour traiter la transpiration humaine et éventuellement les odeurs corporelles, consistant à appliquer sur la surface de la peau une quantité efficace d'une composition telle que définie selon l'une quelconque des revendications 1 à 15.
